(19)

Europäisches
Patentamt
European
Patent Office
Office européen
des brevets

(11) **EP 1 990 402 A1**

(12) **EUROPEAN PATENT APPLICATION**

(43) Date of publication:
**12.11.2008 Bulletin 2008/46**

(51) Int Cl.:
***C12M 3/00*** *(2006.01)*

(21) Application number: **07009153.3**

(22) Date of filing: **07.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **ETH Zürich**
**8092 Zürich (CH)**

(72) Inventors:
 • **Stemmer, Andreas**
 **4132 Muttenz (CH)**

 • **Aschwanden, Manuel**
 **6469 Haldi (CH)**
 • **Franco-Obregon, Alfredo**
 **8051 Zürich (CH)**
 • **Rey, Antje Gabriele**
 **8335 Hittnau (CH)**
 • **Vonderheit, Andreas**
 **8049 Zürich (CH)**
 • **Enning, Raoul**
 **8032 Zürich (CH)**
 • **Sakai, Miho**
 **8050 Zürich (CH)**

(54) **Bioreactor to apply mechanical forces as an anabolic stimulus**

(57)     The current invention describes a bioreactor (1) which could be used in the field of cytomechanics. The bioreactor (1) comprises an electro-active polymer actu- ator (2), also known as artificial muscle, which is bonded to a deformable, water impermeable biocompatible sub- strate/matrix (8, 9). This layered structure is the flexible membrane of the bioreactor (1).

Fig. 1

EP 1 990 402 A1

## Description

## FIELD OF THE INVENTION

The invention lies in the field of bioreactors used in biomedical science, especially in the field of cytomechanics.

## BACKGROUND OF THE INVENTION

It has been well-accepted that mechanical forces serve as an anabolic stimulus for cells. The tools for studying this process, although highly anticipated, have been slow in coming. Specifically, the development programs of all structural tissues, such as bones, muscles and convenient tissues, are modulated by mechanical stimuli. The study of these stimuli and their respective responses has spawned a field known as Mechanobiology. In essence, mechanical forces mold our tissues. Moreover, the development programs and normal functioning of all tissues that respond to mechanical stimuli, such as blood vessels, skin, and nervous tissue, will also be influenced by mechanical forces. Unfortunately, the interaction between mechanical stimuli and functional tissue development has not been adequately studied and optimized due to the technological shortcomings of currently available test apparatuses.

US5348879, by Shapiro et al., was published in 1994 and discloses a method for imparting biaxial mechanical forces to cell cultures. A membrane, upon which a living cell culture is arranged, is actuated mechanically. A displacement applicator driven by a motor, which is interconnected to the membrane, is moving up and down, thereby stretching the membrane. Stretching of the membrane imparts biaxial mechanical forces to the cells. Due to the complicated mechanical activation of the strain force and the motor incorporated, the size of the device can not be minaturized. Also, the heat resulting from the motor has a negative influence for the living cells, since only a small increase of the temperature of the cell environment may have an impact on essential enzymes. Furthermore, the activation mechanism requires regular lubrication in order to reduce friction between the components, especially between the displacement applicator and the membrane.

US5153136, by Vandenburgh, was published in 1992 and describes a relatively complicated apparatus for growing tissue specimens in vitro. The tissue specimen is situated in a compartment on an expandable membrane. A step motor is moving a plate with various pins - each pin for one compartment - up and down. The pins are in contact with the membrane and stretch it according to their movement. The step motor is connected to a controller, which allows to apply an activity pattern to the membrane in form of a continuous stretch or a repetitive stretch and release. Beside the fact, that the mechanism is complicated and difficult to maintain (cleaning, lubrication), it is impossible to minaturize it.

US4851354, by Winston et al., published in 1989, describes an apparatus for studying cells in a culture under conditions which should reproduce their natural, in vivo mechanical environment. The device comprises an airtight well having an optically transparent base of a biologically compatible material and an optically transparent, removable cap. Cells may be grown on the biological compatible material. Beneath the base and coupled to it is a airtight reservoir with an optically transparent base. The reservoir can be filled with a pressurized media to create a deformation of the compliant base thereby exerting biaxial strain on the cells attached thereto. One difficulty results from the need to arrange the biocompatible material in an airtight manner. The device seams not to be appropriate to apply unidirectional strain.

US6048723, by Banes, was published in 2000 and relates to a device for culturing cells. The device comprises a flexible membrane arranged between a base and a body each having a plurality of mutually aligned openings. When a pressure is applied to the underside of the membrane, the membrane moves vertically up and down and thereby stretches, inducing strain on the cells cultured thereon. One disadvantage consists in the relatively complicated setup of the device. A further disadvantage results from the up and down movement when pressure is applied. Thereby it is e.g. not possible to monitor the cells during deformation, because they move out of focus due to the vertical movement of the membrane.

US6998265, by Banes, was published in 2002 and describes an apparatus to grow cell cultures. Interconnected to a flexible membrane is an anchor with an anchor stem to which cells may be attached. A method for growing and mechanically conditioning three-dimensional cell constructs begins by drawing the flexible membrane and the anchor stem into a cavity. Cells are then supplied and allowed to attach to the anchor stem. The flexible membrane is released from within the cavity, resulting in a three-dimensional structure of cells attached to the anchor stem. Various types of strain for mechanical conditioning of the cells can be applied to the structure. Since the mechanism is based on a vacuum to draw the membrane into the cavity it is restricted to low frequency cycling. A further draw-back results from the vertical movement of the cells during operation of the unit. Thereby making it impossible to study the cell behaviour during application of strain.

State of the art bioreactors are not only complicated in their setup, but also limited in their spatial and temporal resolution, and size. One shortcoming of currently available technologies is that the basic unit, responsible for mechanical motion, is often based on conventional motors that have low electromechanical conversion efficiency and therefore, large heat dissipation. It is known that about 5 C° increase in temperature may inactivate essential enzymes and commences to denature (unfold) proteins. This limits the use of such bioreactors in the commercially available incubators that normally do not come equipped with cooling mechanisms. The large-scale machines also do not allow an easy handling of the culture dishes because the cells are grown on membranes that are fixed directly to this bulky bioreactor, requiring a serious (and for most lab often undesirable) modification of the cell incubator. Other disadvantages include a stretch magnitude that is too low, stretch magnitude to frequency response relation that is much smaller than required, and lubrication methods that make the entire setup somewhat dirty and subject to contamination. A further disadvantage of most devices results from the immanent vertical, out of plain movement of the cells during application of strain. Thereby making it impossible to optically survey the samples during application of strain.

One commercial device of Flex Cell International, uses vacuum (vacuum grease required) to deform a flexible membrane of biocompatible properties. This method yields two-dimensional substrate elongation of up to 18 % (30 % unidirectional). The flexible membrane is actuated by an external vacuum pump, which is connected with a tube to the cell culture dishes inside of an incubator.

Although this system is used in many research laboratories, it has the major drawback of limited temporal and spatial resolution. The vacuum pump is limited to 5 Hz (at very low amplitudes, much less than 10% strain). At a frequency of 1 Hz the maximal available elongation drops dramatically to 12 %. Additionally, the thin membrane, on which cells are grown, is moved over a loading post. This causes friction, which weakens the membrane, requires lubrication, and introduces local heating of the membrane at high drive frequencies. Therefore, the system is limited to low frequency experiments in which the directionality of distension is not an issue.

## SUMMARY OF THE INVENTION

It is an object of the present invention to overcome the disadvantages of the devices known from prior art.

The current invention describes a bioreactor which can be used in the field of cytomechanics.

A bioreactor according to the invention in general comprises a layered structure with an electroactive actuator mechanically interconnected to a deformable biocompatible substrate/matrix suitable to receive a specimen. The deformable biocompatible substrate/matrix may be elastically deformable arranged, e.g. in a prestretched manner in a supporting frame, such that unwanted out of plane deformation can be avoided. The bioreactor allows the application of mechanical strain as an anabolic stimulus to cell structures of a specimen in plane of the cell structure.

One embodiment of the invention discloses a method which is based on electroactive polymer actuators (EAP) to apply well-defined strain and thereby stress and conformational changes onto cell substrates with a precision not achievable with existing technologies. EAP based bioreactors according to the present invention offer a frequency response and spatial resolution far beyond currently available methods. In addition, they can mediate all modalities of mechanical stress by easily actuating different regions of the EAP.

Electroactive polymer (EAP) actuators consist of materials that employ voltage, field, light, or temperature driven dimensional changes to produce forces and displacements. Such materials are e.g. dielectric elastomers, ferroelectric polymers, liquid crystal elastomers, thermal and ferroelectric shape memory alloys, ionic polymers/metal composites, conducting polymers, or carbon nanotubes. A good overview about actuators currently available can be found under the following internet address: http://www.actuatorweb.org.

In an embodiment of the present invention dielectric elastomer actuators are used to implement an electrically tunable substrate on which biological systems/tissues can be grown under mechanical deformation (shear, stretch, compression) applied in the plane of the substrate. Although, primarily implementations based on dielectric elastomer actuators are discussed in more detail, depending on the field of application, implementations based on other types of EAP actuators may also be possible and are therefore not in general excluded from the principle idea of this invention. One advantage consists in that it becomes possible to mechanically activate cell substrates in three dimensions (3D) for tissue engineering, e.g. it becomes possible to apply strain in three dimensions.

Beside dielectric elastomer actuators, as described in more detail further down, ionic polymer/metal composites (IPMC) may also be advantageous for certain fields of application, due to their operation at low voltage and in liquids. A ionic polymer-metal composite (IMPC) consists in general out of a polymer electrolyte arranged between two electrodes. Deflection of the layered structure towards one of the electrodes occurs as a result of a field induced change in ion concentration, which attracts water molecules (or other solvents) to one side of the polymer. The non-uniform distribution of water produces swelling of one side of the actuator and contraction of the other, generating torque on the member and a bending motion.

Further embodiments may be based on conducting polymers. Conducting polymers are in general electrical conducting organic materials featuring conjugated structures. Electrochemical changed oxidation states leads to the addition or removal of charge from the polymer backbone and a flux of ions to balance charge such that swelling or contraction of the material occurs by insertion of ions between polymer chains.

An embodiment of a bioreactor according to the present invention in general comprises a single- or a multi-layered elastically deformable membrane (film) on which cells of a specimen may be grown. The elastically deformable membrane is normally arranged attached to a supporting means, e.g. a frame, and is actuated directly or indirectly by a mechanically interconnected actuator as previously mentioned. In certain embodiments the elastic film may have a three-dimensional structure, e.g. in the form of a tube. Strain is applied to the cells by stretching and relaxing of the membrane in a periodic manner via the dielectric actuator.

An embodiment of a bioreactor according to the invention comprises an elastic film arranged between at least two opposite electrodes. The elastic film normally is arranged in a prestretched manner. At least one layer of biocompatible material suitable to receive a specimen is mechanically interconnected to one side of the elastic film and the electrodes. When a voltage is applied to the electrodes a local compression of the elastic film arranged between the electrodes occurs due to coulomb-forces (primary deformation). Due to the poison-ratio of the material of the elastic film a lateral expansion of the elastic film arranged between the electrodes takes place (secondary deformation). When the elastic film is arranged in a prestretched manner the material of the elastic film adjacent to the electrodes contracts in plane (tertiary deformation) due to the lateral expansion of the material arranged between the electrodes. Either the secondary and/or the tertiary movement are transferred via the interconnected layer of biocompatible material to a specimen. Good results are achieved in that the elastic film is arranged in a supporting frame. Depending on the field of application, the supporting frame has a central square or round opening in which the elastic film is arranged. Other 2- or 3-dimensional shapes, e.g. such as tubular structures, may be suitable.

If appropriate the elastic film is supported from behind, opposite to the specimen, e.g. by a rigid surface or a fluid. To prevent corrosion a layer made out of water impermeable material is arranged between the elastic film and at least one electrode and the layer of biocompatible material. Depending on the type of strain to be achieved, the biocompatible material suitable to receive the specimen is arranged on top of an electrode where its deformation is related to the secondary deformation which occurs in that area. Alternatively or in addition the biocompatible material is arranged adjacent to electrodes where the occurring strain is related to the tertiary deformation of that area.

If the membrane is arranged in a prestretched manner out of plane deformation can be prevented without additional support. The electrodes are preferably made out of an easily deformable (stretchable) material such that no hindering of the lateral in-plane deformation results. Depending on the field of application the cells may be arranged in the area of the electrodes or in an electrode-free area. Thereby different stretching of the cells is achieved. The shape of the electrodes and the supporting frame determine the type of deformation, e.g. unilateral or multi-directive. In that more than one pair of electrodes are arranged it is possible to determine flexibly the type of strain applied to the specimen. E.g. it is possible to arrange pairs of opposite electrodes at angles of 60°, 90°, 120° or 180° apart from each other. Other angle combinations may be appropriate. Thereby it becomes possible to apply strain and shear or combinations thereof in different directions in that the pairs of electrodes are driven with different voltage out of appropriate voltage supplies.

An embodiment of a bioreactor according to the invention preferably comprises a dielectric elastomer actuator, that when activated in a controlled manner by a voltage supply, causes a lateral deformation of an mechanically interconnected biocompatible substrate layer, on which cells of a specimen can be grown under mechanical actuation. Dielectric elastomer actuators are used in this implementation because they are simple and cheap to manufacture, can generate large strain (20 % - 380 %) and moderate stress (several MPa peak), they have large work density and moderate to high frequency bandwidth (10 Hz to > 1 kHz).

Dielectric elastomer actuators are based on electrostatic attraction between two conductive layers (electrodes) applied opposite to each other on the surface of an elastically deformable film. The elastic film is normally arranged in a prestretched manner. When a voltage V is applied to the compliant electrodes, an electromechanical thickness strain

$$s_z = -p/Y = -\varepsilon\varepsilon_0 \frac{V^2}{Yt^2}$$

is induced by electrostatic forces. Here, t represents the thickness, Y is the modulus of elasticity (Young's modulus), $\varepsilon_0$ denotes the permittivity of free space and $\varepsilon$ is the dielectric constant of the acrylic elastomer (e.g. $\varepsilon_{VHB}$=3.21). Assuming that the volume of the elastic film in general remains constant (1+sx)(1+sy)(1+sz) = 1 (Poisson's ratio = 0.5) and that the strain in all planar directions is equal, the following equation results

$$s_{planar} = \frac{1}{\sqrt{1 - \varepsilon\varepsilon_0 \frac{V^2}{Yt^2}}} - 1.$$

Therefore, when the actuator is driven by an interconnected voltage supply, the resulting strain is transmitted directly or indirectly to a mechanically bonded biocompatible layer on which biological specimens such as cells can be grown. Thus, a controlled mechanical stress can be induced onto cells by simply changing the voltage applied to the EAP actuator. The biocompatible layer can be a permanently bonded layer or an exchangeable layer (i.e. heat sensitive glue). The exchangeability of the membrane offers the advantage, that multiple experiments can be conducted with the same bioreactor.

Making of a bioreactor according to the invention in general comprises the following steps:

1. Stretching of a dielectric elastic film;

2. Applying at least one pair of opposite electrodes, e.g. by contact printing of carbon black;

3. If appropriate stretching the dielectric elastic film along with electrodes;

4. Applying the dielectric elastic film in a prestretched manner on a holding frame;

5. Sealing the electrodes with a water impermeable elastic layer (e.g. acrylic VHB material by 3M).

6. Applying of a flexible membrane onto the previous layers suitable to receive a vivid specimen, e.g. by spin coating or appropriate methods;

7. Adding a cell adhesive protein to the coated membrane or modifying the same membrane to achieve bio-compatibility for a life specimen.

A prototype of a bioreactor according to the present invention which has been tested in biological experiments in an incubator (at 37 °C, relative humidity >95% and 5 % $CO_2$), was made as follows:

1. An acrylic elastic film, in the present example VHB 4910 made by the company 3M, was stretched by 300 % x 300 %;

2. Electrodes, in this example made out of Carbon Black powder, were contact-printed onto both sides of the film opposite to each other;

3. The acrylic elastic film was then fixed in the stretched manner on a bottom part of a supporting frame;

4. A water impermeable layer, in the present example made out of VHB 9460, was also stretched by e.g. 300 % by 300 % and then bonded onto the acrylic elastic film and the upper electrode;

5. On the top side a further frame made out of PMMA was fixed onto the bottom part of the frame, fixing the stretched layers in between;

6. On each side, metal contacts were mounted to the frame and contacted to the electrodes;

7. A biocompatible substrate layer was applied on to the water impermeable layer by spin coating (good results were achieved by applying a layer of PDMS Sylgard 184 of Dow Corning with a thickness of 15 $\mu$m);

8. Furthermore, the device was undertaken an oxygen plasma treatment to change the PDMS surface property from hydrophobic to hydrophilic.

An EAP-based bioreactor according to the present invention may achieve linear strains in the range of 10 % at frequencies in the range of 10 Hz. Depending on the embodiment other values may be achieved. Additionally, the bioreactor based on electroactive polymers can be designed to produce linear or bi- or multi-axial strains of substrates measuring approximately 100 $\mu$m by 100 $\mu$m up to several square centimetres. Therefore, the device e.g. allows in vitro studies of mechanical shock experiments that were not possible before. The device normally does not generate heat by friction and, therefore, long-run studies are possible.

A bioreactor according to the herein disclosed invention in general is driven by an appropriate signal generator which is interconnected to the electrodes to apply a voltage signal of sufficient height. Due to the reason that no large current is necessary to drive the device, only two, relatively small electrical cables need to be attached. Electrical signals are more stable (unaffected by length of cable) than vacuum lines as known from the prior art. For instance, slight pinching will not influence the ability of an electrical cable to carry current, but can reduce vacuum pressure and frequency response in an indeterminable manner. Therefore, an EAP-bioreactor according to the invention is more accurate and reliable than a vacuum based system. A further advantage is the silent operation compared to mechanical motors and vacuum pumps, additionally the discussed technology can be extended to a three dimensional (3D) system.

In difference to the devices known from prior art, an EAP-actuator according to the invention can e.g. easily fit on the stage of a microscope or within the chamber of a incubator without the need of further modification. Commercially available mechanical stretchers are large and bulky and most microscopes and incubators cannot be used in conjunction with ongoing stretching paradigms.

A further advantage of the invention consists in the transparency of the device. Cells can be examined in real time while undergoing stretch. Most other methods require viewing and stretching to take place separately (in the vacuum method, the loading post is opaque). Alternatively, special (expensive) measures need to be implemented to maintain the specimen within the focal plane of the objective while stretching.

EAP-actuators according to the present invention can mediate all modalities of mechanical stress by specifically pre-straining the EAP layer and/or by strategically positioning the electrodes acting on the EAP layer. In difference to that, prior art mechanical bioreactors need to be reconfigured to apply different stretch modes.

EAP-actuators according to the invention exhibit a more linear spatial and temporal resolution far beyond contemporary means of applying mechanical stretch to cells. EAP-actuators may achieve the same stretch everywhere along the activating electrode. Mechanical stretchers, on the other hand, exhibit a loss in fidelity depending on the distance from the mechanical focus.

EAP-actuators according to the invention in general do not require any lubrication or maintenance, reducing the chance of biological cross contamination when the actuators are handled as well as reducing the chance of contaminating and damaging imaging microscopes.

An EAP-actuator according to the invention may be made flexible, such that it can be deformed into various configurations without altering its ability to produce stretching or compressing forces. This can be used to produce three-dimensional bioreactors, e.g. for tissue engineering purposes.

**BRIEF DESCRIPTION OF THE DRAWINGS**

The herein described invention will be more fully understood from the detailed description given herein below and the accompanying drawings which should not be considered limiting to the invention described in the appended claims. The drawings are showing:

Fig. 1    A first embodiment of a bioreactor according to the present invention in a side view;

Fig. 2    A top view of a specimen and a side view of a bioreactor showing the effects of applying a voltage to the electrodes of the actuator;

Fig. 3    A diagram showing the relation between voltage and strain in a bioreactor;

Fig. 4    A further embodiment according to the present invention showing a three dimensional bioreactor;

Fig. 5    A further embodiment of a bioreactor in a side view.

**DESCRIPTION OF EMBODIMENTS**

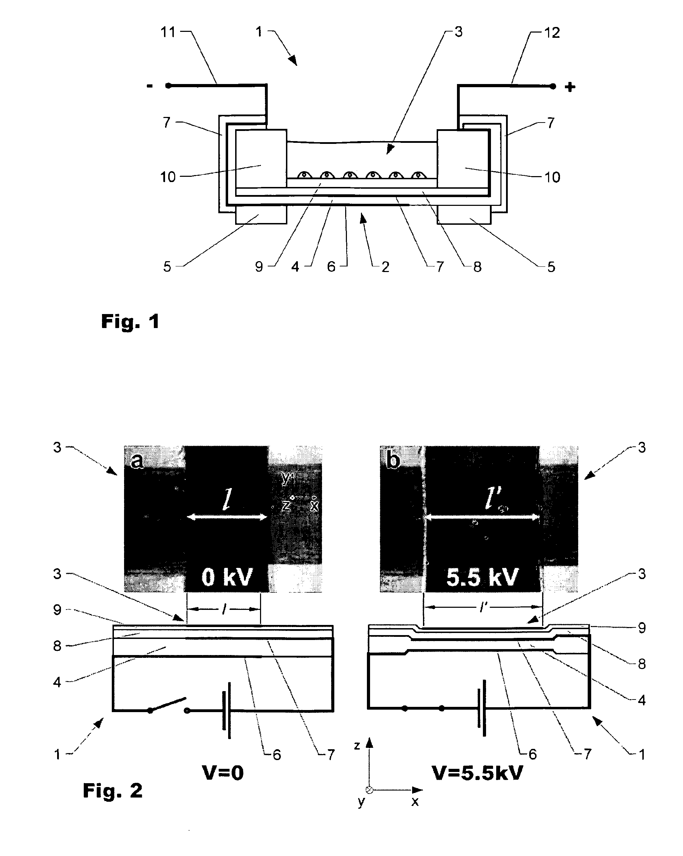

**Figure 1** schematically shows a first embodiment of a bioreactor 1 according to the present invention in a side view. The bioreactor 1 comprises a mechanical actuator 2 to apply strain to a specimen 3 based on the mechanical deformation of a first elastic film 4.

In the shown embodiment the first elastic film 4 is stretched uniformly to a certain amount e.g. 100% to 500% and then fixed in a prestretched manner on a frame 5. The frame 5 may be made out of several materials, such as polymers, e.g. polymethyl methacrylate (PMMA), or ceramics, glass or metal. The first elastic film 4 may be made out of different materials, good results are achieved by elastomer materials such as silicones or acrylic polymers. The first elastic film 4 preferably has a thickness in the range of 1 $\mu$m to 100 $\mu$m. Depending on the field of application other values may be appropriate.

On the upper and the lower side of the first elastic film 4 two opposite, compliant electrodes 6, 7 are applied to the first elastic film 4, e.g. by contact printing of carbon black. Other methods may be appropriate. On the upper side of the first elastic film 4 and the upper electrode 7 a second film 8 made out of a deformable material is attached and mechanically interconnected such that deformation of the first elastic film 4 is transmitted to the second film 8. In general the second film 8 is made out of a water impermeable, elastic material such as an acrylic polymer (e.g. VHB9460) to prevent corrosion of the electrodes 6, 7. On top of the second elastic film 8 a biocompatible layer 9 is arranged suitable to receive the specimen 3.

The biocompatible layer 9 may be surrounded by a housing 10 which is normally sealingly interconnected to the second film 8. Thereby it becomes possible to operate the inside of the bioreactor under wet or other special conditions.

In the shown embodiment the electrodes 6, 7 are contacted by metallic connectors 11, 12 to connect the electrodes to a voltage supply (not shown in detail) to drive the actuator 2 and thereby to mechanically stimulate the cell structure (specimen) 3. The bioreactor 1 can e.g. be used for in vitro experiments of biological systems 3.

**Figure 2** shows in a simplified manner the principle of a bioreactor 1 according to the invention. In the upper parts of **Figures 2a** and **2b** a picture of a biological specimen 3 is visible and in the lower part the setup of the bioreactor 1 is schematically shown in a side view. While in **Figure 2a** no voltage, in **Figure 2b** a voltage of 5.5kV is applied to drive the actuator 2 and to stretch the specimen 3 in x-direction.

After applying of a voltage of 5.5kV, the electrodes 6, 7 attract each other by coulomb forces thereby reducing the thickness (z-axis) of the elastic film 4 in the area between the electrodes 6, 7. Due to the compression of the material between the electrodes a lateral expansion in x-direction takes place by reason of the poisson ratio of the material.

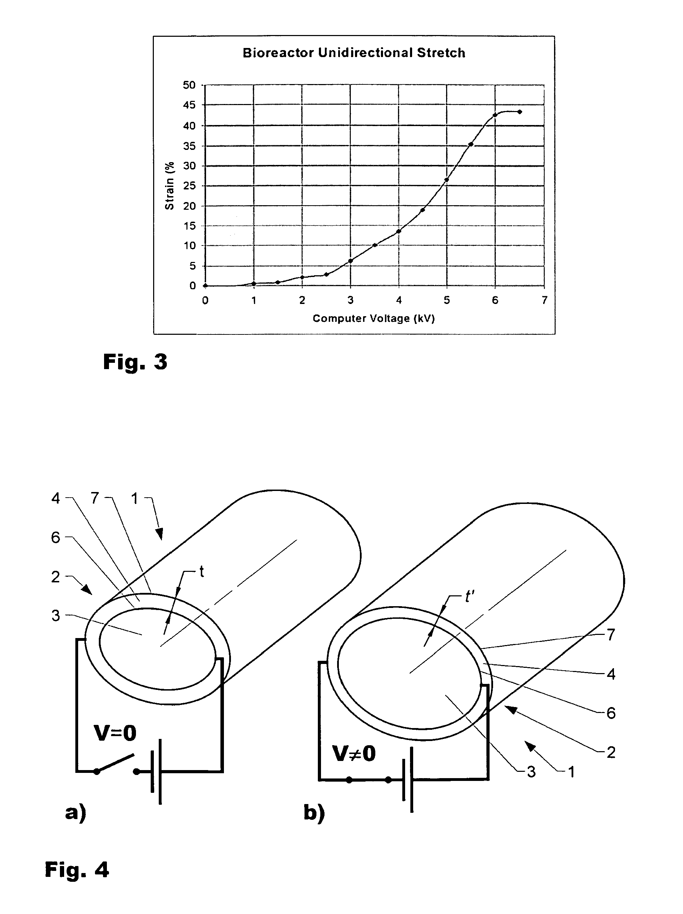

**Figure 3** shows the dependency between voltage (kV) and strain (%) of an actuator 2 of a bioreactor 1 as shown in Figure 1. As it can be seen, strain of up to 43 % can be achieved when a voltage of 6.5 kV is applied. Further measurements have shown that the frequency response is in the range of 10 Hz when the bioreactor is driven in strain ranges, which are used in biological experiments (5 - 10 %). This is at least an order of magnitude higher than what is possible with previous bioreactors.

**Figure 4** shows a further embodiment of a bioreactor 1 according to the present invention with multi-dimensional deformation of a specimen 3. A here tubular dielectric elastomer actuator 2 which comprises an internal 6 and an external electrode 7 encompassing a film of elastomer material 4. If appropriate the film layer 4 may be prestretched. Biological material/matrix (specimen) 3 is enclosed in the tubular bioreactor 1. In **Figure 4a** no voltage (V=0) is applied to the electrodes 6, 7 such that the elastic film 4 is not compressed (no change in thickness t of the layer). When a voltage (V≠0) is applied to the electrodes 6, 7 as shown in **Figure 4b**, the elastic film 4 of the dielectric elastomer actuator 2 is compressed due to coulomb forces such that the thickness t changes to t' and thereby the actuator expands three dimensionally. If appropriate other types of EAP-actuators may be used.

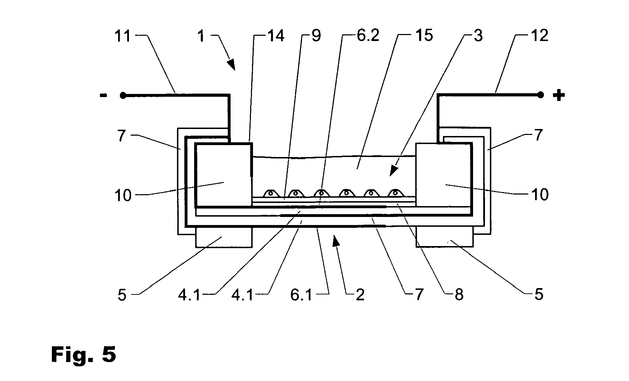

**Figure 5** shows a further embodiment of a bioreactor which in general corresponds to the embodiment as shown in Figure 1. Similar items are marked with the same numbers. For the general description it is referred to Figure 1.

In the embodiment according to Figure 5, the actuator 2 has a multi-layered setup with an upper and a lower elastic film 4.1, 4.2 and three electrodes 6.1, 6.2, 7 arranged congruent to each other. One first electrode 7 is arranged between the first and the second layer 4.1, 4.2 and the second and the third electrode 6.1, 6.2 are arranged opposite to each other and the first electrode 7 on top of the upper film 4.2 and underneath the second film 4.1. Due to the double pair arrangement of the electrodes 6.1, 6.2, 7 it becomes possible to apply more force to compress the elastic films 4.1, 4.2. A further advantage consists in that the electrodes 6.1, 6.2 can be hold on ground potential and thereby may act as shielding layer to reduce electric fields. In the shown embodiment the upper electrode 6.2 (electrode 14) reaches partially into a liquid 15 covering the specimen 3. Thereby it is achieved that the liquid 15 and the specimen 3 are maintained on an equivalent electrical potential as electrode 11.

**Claims**

1.  Bioreactor (1) comprising an electroactive polymer actuator (2) mechanically interconnected to a deformable layer of biocompatible material (9) suitable to receive a specimen (3) such that a deflection of the electroactive polymer actuator (2) results in a thereto related deformation of the layer of biocompatible material (9), such that strain is applied to the specimen (3).

2.  The bioreactor (1) according to claim 1, **characterized in that** the electroactive polymer actuator (2) is one out of the group of the following electroactive polymer actuators: dielectric elastomers, ferroelectric polymers, liquid crystal elastomers, thermal and ferroelectric shape memory alloys, ionic polymers/metal composites, conducting polymers, or carbon nanotubes.

3.  The bioreactor (1) according to one of the preceeding claims, **characterized in that** the electroactive polymer actuator (2) comprises an elastic film (4) arranged between two opposite electrodes (6, 7) and the layer of biocompatible material (9) is arranged adjacent to the elastic film (4) such that a lateral deformation of the elastic film (4) is transmitted to the layer of biocompatible material (9).

4.  The bioreactor (1) according to claim 3, **characterized in that** the biocompatible material (9) is arranged at least partially laterally along the elastic film (4).

5.  The bioreactor (1) according to claim 3, **characterized in that** the biocompatible material (9) is arranged in plane (x, y) adjacent to the elastic film (4).

6.  The bioreactor (1) according to claim 4 or 5, **characterized in that** the elastic film (4) is arranged between two electrodes (6, 6.1, 6.2, 7) arranged in general opposite to each other and at least one layer of biocompatible material (9) suitable to receive a specimen (3) is mechanically interconnected to the elastic film (4) such that a compression (z) of the elastic film (4) by the electrodes (6, 7) when applying a voltage results in a lateral deformation (x, y) of the elastic film (4) due to the poisson-ratio of the material of the elastic film (4) and the thereto interconnected layer of biocompatible material (9).

7.  The bioreactor (1) according to claim 6, **characterized in that** the actuator (2) comprises at least three electrodes (6, 6.1, 6.2, 7) which are arranged congruent to each other on opposite sides of two elastic films (4.1, 4.2).

8.  The bioreactor (1) according to one of the previous claims, **characterized in that** a layer (8) made out of water impermeable material is arranged between the elastic film (4) and at least one electrode (6, 7) and the layer (9) of biocompatible material.

9.  The bioreactor (1) according to one of the previous claims, **characterized in that** the biocompatible layer (9) of material is arranged on top of an electrode (6, 7).

10. The bioreactor (1) according to one of the previous claims, **characterized in that** the layer of biocompatible material (9) is arranged beside at least one electrode (6, 7).

11. The bioreactor (1) according to one of the previous claims, **characterized in that** the elastic film (4) is arranged between more than one pair of opposite electrodes (6, 7).

**12.** The bioreactor (1) according to claim 11, **characterized in that** pairs of electrodes (6, 7) are arranged with respect to a central portion at an angle of 60°, 90°, 120° or 180° with respect to each other.

**13.** The bioreactor (1) according to one of the previous claims, **characterized in that** at least the elastic film (4) and the layer (9) of biocompatible material are translucent such that a specimen (3) can be iluminated and/or examined from the back.

**14.** The bioreactor (1) according to one of the preceding claims, **characterized in that** the electroactive polymer actuator (2) has a tubular shape.

**15.** The bioreactor (1) according to claim 14, **characterized in that** the layer of biocompatible material (9) is arranged inside the tubular structure.

**16.** The bioreactor (1) according to one of the claims 1 to 13, **characterized in that** the bioreactor (1) comprises a supporting frame (5) for the elastic film (4).

**17.** The bioreactor (1) according to claim 14, **characterized in that** the elastic film (4) is arranged in a prestretched manner in the supporting frame (5).

**18.** The bioreactor (1) according to one of the claims 14 or 17, **characterized in that** the supporting frame has a central opening in which the elastic film is arranged.

**19.** Method of manufacturing a bioreactor (1) according to one of the previous claims, comprising the following steps:

    a) stretching of an elastic film (4);
    b) interconnecting of at least one pair of opposite electrodes (6, 7) to the elastic film (4);
    c) attaching the elastic film (4) on a supporting frame (5);
    d) if appropriate applying a layer (8) of water impermeable material onto the elastic film (4) and at least one electrode (6, 7);
    e) applying a layer (9) of biocompatible material onto the elastic film (4) and/or the layer (8) of water impermeable material.

**Fig. 1**

**Fig. 2**

## Bioreactor Unidirectional Stretch

**Fig. 3**

a)

**V=0**

b)

**V≠0**

**Fig. 4**

**Fig. 5**

**European Patent Office**

**EUROPEAN SEARCH REPORT**

Application Number

EP 07 00 9153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/012071 A (US GOVERNMENT [US]; TABOAS JUAN M [US]; TUAN ROCKY S [US]; HUDSON STEV) 25 January 2007 (2007-01-25) * page 5, line 16 - page 7, line 14 * ----- | 1 | INV. C12M3/00 |
| A | US 2004/005297 A1 (CONNELLY PATRICK R [US] ET AL) 8 January 2004 (2004-01-08) * sentence 74 - sentence 78 * ----- | 1,18 | |
| A | US 6 190 893 B1 (SHASTRI VENKATRAM R [US] ET AL) 20 February 2001 (2001-02-20) * claims 1,2 * ----- | 1,18 | |
| A | WO 2005/123905 A (NAKATA KEN [JP]) 29 December 2005 (2005-12-29) * abstract * ----- | 1 | |
| A | US 2006/094112 A1 (BABALOLA OMOTUNDE [US] ET AL) 4 May 2006 (2006-05-04) * claim 1 * ----- | 1 | |
| A | DE 101 51 822 A1 (HEUBACH JUERGEN [DE]; INST POLYMERFORSCHUNG DRESDEN [DE]) 15 May 2003 (2003-05-15) * claim 1 * ----- | 1,18 | TECHNICAL FIELDS SEARCHED (IPC) C12M |

The present search report has been drawn up for all claims

| Place of search | Date of completion of the search | Examiner |
|---|---|---|
| Berlin | 30 August 2007 | Clement, Jean-Paul |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.................................................................................................

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 07 00 9153

This annex lists the patent family members relating to the patent documents cited in the above-mentioned European search report.
The members are as contained in the European Patent Office EDP file on
The European Patent Office is in no way liable for these particulars which are merely given for the purpose of information.

30-08-2007

| Patent document cited in search report | | Publication date | Patent family member(s) | | Publication date |
|---|---|---|---|---|---|
| WO 2007012071 | A | 25-01-2007 | NONE | | |
| US 2004005297 | A1 | 08-01-2004 | NONE | | |
| US 6190893 | B1 | 20-02-2001 | WO 0017322 A2 | | 30-03-2000 |
| | | | US 2002034796 A1 | | 21-03-2002 |
| WO 2005123905 | A | 29-12-2005 | EP 1780266 A1 | | 02-05-2007 |
| US 2006094112 | A1 | 04-05-2006 | NONE | | |
| DE 10151822 | A1 | 15-05-2003 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5348879 A, Shapiro
- US 5153136 A, Vandenburgh
- US 4851354 A, Winston
- US 6048723 A, Banes
- US 6998265 B, Banes